Europäisches Patentamt

European Patent Office

Office européen des brevets

(11) Numéro de publication : **0 382 620 B1**

# (12) FASCICULE DE BREVET EUROPEEN

(45) Date de publication du fascicule du brevet :
**20.07.94 Bulletin 94/29**

(51) Int. Cl.⁵ : **G02C 7/04**, A61F 2/14, A61F 2/16

(21) Numéro de dépôt : **90400309.2**

(22) Date de dépôt : **05.02.90**

(54) **Lentille ophtalmique diffractive pour la correction de l'astigmatisme.**

(30) Priorité : **06.02.89 FR 8901472**

(43) Date de publication de la demande :
**16.08.90 Bulletin 90/33**

(45) Mention de la délivrance du brevet :
**20.07.94 Bulletin 94/29**

(84) Etats contractants désignés :
**CH DE ES GB IT LI NL**

(56) Documents cités :
**EP-A- 0 064 812
EP-A- 0 109 753
GB-A- 2 185 126
US-A- 4 210 391
US-A- 4 508 436**

(72) Inventeur : **Baude, Dominique
1, allée du 8 Mai 1945
F-93400 Saint-Ouen (FR)**
Inventeur : **Chavel, Pierre
6, Avenue de Rocroi,
Le Bois des Ormes
F-91380 Chilly Mazarin (FR)**
Inventeur : **Joyeux, Denis
51, rue des Chardonnerets
F-91940 Les Ulis (FR)**
Inventeur : **Taboury, Jean
89, rue Houdan
F-92230 Sceaux (FR)**

(74) Mandataire : **Martin, Jean-Jacques et al
Cabinet REGIMBEAU
26, Avenue Kléber
F-75116 Paris (FR)**

(73) Titulaire : **ESSILOR INTERNATIONAL, Cie
Générale d'Optique
1 Rue Thomas Edison,
Echat 902
F-94028 Créteil Cédex (FR)**

EP 0 382 620 B1

## Description

La présente invention concerne une lentille optique destinée à assurer la correction de l'astigmatisme.

Il peut s'agir aussi bien d'une lentille de contact que d'un implant intra-oculaire ou que d'une lentille intra-cornénne.

L'astigmatisme est un défaut de l'oeil qui a pour conséquence de donner d'une source ponctuelle située à l'infini une image composée de deux petits segments lumineux perpendiculaires et espacés l'un de l'autre le long de l'axe optique d'une distance fonction du degré d'astigmatisme. Ces deux segments correspondant aux focales du faisceau astigmate et peuvent être situés du même côté de la rétine en avant (astigmatisme myopique) ou en arrière (astigmatisme hyperopique) de celle-ci, ou de part et d'autre de la rétine (astigmatisme mixte). Ces défauts peuvent avoir deux origines, soit cornéenne soit cristallinienne, la cornée et le cristallin présentant des surfaces qui ne sont pas de révolution et qui comportent deux rayons principaux.

La correction de l'astigmatisme de l'oeil est obtenue en compensant cette aberration, afin d'obtenir pour un objet ponctuel une onde sphérique centrée sur le point image au niveau de la rétine.

Cette correction est obtenue jusqu'ici à l'aide de lentilles sphérocylindriques qui correspondant fonctionnellement à l'association d'une lentille sphérique et d'une lentille cylindrique.

Ces lentilles ont déjà rendu de grands services. Cependant, la définition précise des surfaces sphérocylindriques aptes à générer une correction optimale reste souvent délicate et complexe.

Les spécialistes ont envisagé depuis quelques années de développer des lentilles destinées à compenser la presbytie, comprenant des composants diffractifs (voir par exemple EP-A-0 064 812, US-A-4 637 697, EP-A- 0 109 753 ou encore les demandes de brevet FR-88 06 699 déposée le 19 mai 1988 et FR-88 14 634 déposée le 9 Novembre 1988 au nom de la Demanderesse).

A cet effet, les lentilles diffractives jusqu'ici proposées comprennent une série de structures diffractives concentriques autour de l'axe optique de la lentille.

La présente invention vient maintenant proposer de nouveaux moyens, utilisant des composants diffractifs, destinés à corriger l'astigmatisme de l'oeil.

Les lentilles diffractives ophtalmiques conformes à la présente invention sont caractérisées à cet effet par le fait qu'elles comprennent des composants diffractifs dont le contour est délimité par des courbes coniques à centre non dégénérées, du type hyperbolique ou ellipse, à l'exclusion des cercles.

Dans le cadre de la présente demande de brevet, le terme "contour" désigne la frontière séparant deux zones adjacentes diffractives.

Plus précisément, selon l'invention, la lentille comprend avantageusement un ensemble de zones diffractives adjacentes, de contour hyperbolique ou elliptique dont la périodicité en $r^2$ dans deux directions X et Y, orthogonales entre elles,.centrées sur l'axe de la lentille et coïncidant avec les axes principaux $r_x$, $r_y$ des hyperboles ou ellipses, est déterminée respectivement par les relations :

$$\Delta r^2_x = 2\lambda \, |f_x|$$
$$\Delta r^2_y = 2\lambda \, |f_y|$$

dans lesquelles :

$\Delta r^2 x$ représente la périodicité en $r^2$ des composants diffractifs dans le plan X, (différence entre le $(n+1)^{ème}$ rayon et le $n^{ème}$ rayon en X)

$\Delta r^2 y$ représente la périodicité en $r^2$ des composants diffractifs dans le plan Y, (différence entre le $(n+1)^{ème}$ rayon et le $n^{ème}$ rayon en Y),

$\lambda$ représente la longueur d'onde moyenne d'utilisation,

$f_x$ représente la focale recherchée dans la direction X, et

$f_y$ représente la focale recherchée dans la direction Y.

La Demanderesse a déterminé qu'une telle lentille pourvue d'une structure diffractive permet d'obtenir la même correction de l'astigmatisme qu'une lentille sphéro-cylindrique classique.

D'autre caractéristiques, buts et avantages de la présente invention apparaîtront à la lecture de la description détaillée qui va suivre, et en regard des dessins annexés donnés à titre d'exemples non limitatifs et sur lesquels :

- la figure 1 représente selon une vue schématique en plan, orthogonale à l'axe optique O-O de la lentille, la répartition des zones diffractives de contour elliptique décrivant le composant diffractif d'une lentille optique conforme à la présente invention,

- la figure 2 représente selon une vue similaire schématique en plan, orthogonale à l'axe optique O-O de la lentille, la répartition des zones diffractives de contour hyperbolique décrivant le composant diffractif, d'une autre lentille conforme à la présente invention,

- les figures 3A et 3B représentent le profil de phase, dans deux directions principales X, Y, orthogonales entre elles, de composants diffractifs de contour elliptique, conformes à la présente invention,

2

EP 0 382 620 B1

- les figures 4A et 4B; 5A, 5B ; 6A, 6B ; 7A, 7B ; 8A, 8B représentent deux à deux des vues respectivement similaires aux figures 3A et 3B dans le cadre de variantes de réalisation de composants diffractifs de contour elliptique conformes à la présente invention,
- les figures 9A et 9B représentent le profil de phase, dans deux directions principales X, Y orthogonales entre elles, de composants diffractifs de contour hyperbolique conformes à la présente invention, et
- les figures 10A, 10B ; 11A, 11B ; 12A, 12B ; 13A, 13B ; 14A, 14B ; 15A, 15B ; représentent deux à deux des vues respectivement similaires aux figures 9A et 9B, selon d'autres variantes de réalisation de composants diffractifs de contour hyperbolique conformes à la présente invention.

Comme indiqué précédemment, on a déjà proposé, pour compenser la presbytie, des lentilles diffractives comprenant une série de structures diffractives concentriques, annulaires de révolution autour de l'axe optique de la lentille.

Ces structures diffractives ont généralement toutes le même profil. Elles possèdent une périodicité radiale qui définit l'ensemble des focales potentiellement disponibles selon la relation :

$$f_p = r_o^2/2p\,\lambda$$

où

$r_o^2$     représente le rayon externe de la structure centrale,

p     représente l'ordre de diffraction, et

$\lambda$     représente la longueur d'onde moyenne d'utilisation.

L'efficacité de diffraction des différents ordres est déterminée par le profil de phase de chaque structure.

Pour un composant diffractif de type kinoforme pur avec une variation de phase continue entre 0 et $2\pi$, à la longueur d'onde d'accord l'énergie est diffractée dans un seul ordre, soit l'ordre +1, soit l'ordre -1.

Pour un composant diffractif du type kinoforme échantillonné en n sous zones déphasées de $2\pi/n$, l'ordre de diffraction p = +1 ou p = -1 est le plus intense. Lorsque n = 4, l'efficacité de diffraction à l'ordre p = +1 p = -1 est égale à 0,81 à la longueur d'onde d'accord, alors qu'elle est nulle pour les ordres 0 et -1 ou +1, l'énergie restante se répartissant dans les ordres supérieurs.

Pour un composant diffractif à profil binaire comportant deux sous zones déphasées de $\pi$, les ordres de diffraction p = +1 et p = -1 sont les plus intenses et diffractent chacun 41% de l'énergie indicente, l'ordre 0 étant éteint à la longueur d'onde d'accord.

Alors que les lentilles diffractives jusqu'ici proposées pour compenser la presbytie possèdent des structures diffractives annulaires de révolution autour de l'axe optique de la lentille, la lentille conforme à la présente invention comprend des structures diffractives adjacentes dont le contour n'est plus de révolution autour de l'axe optique, mais est délimité par des courbes coniques à centre non dégénérées.

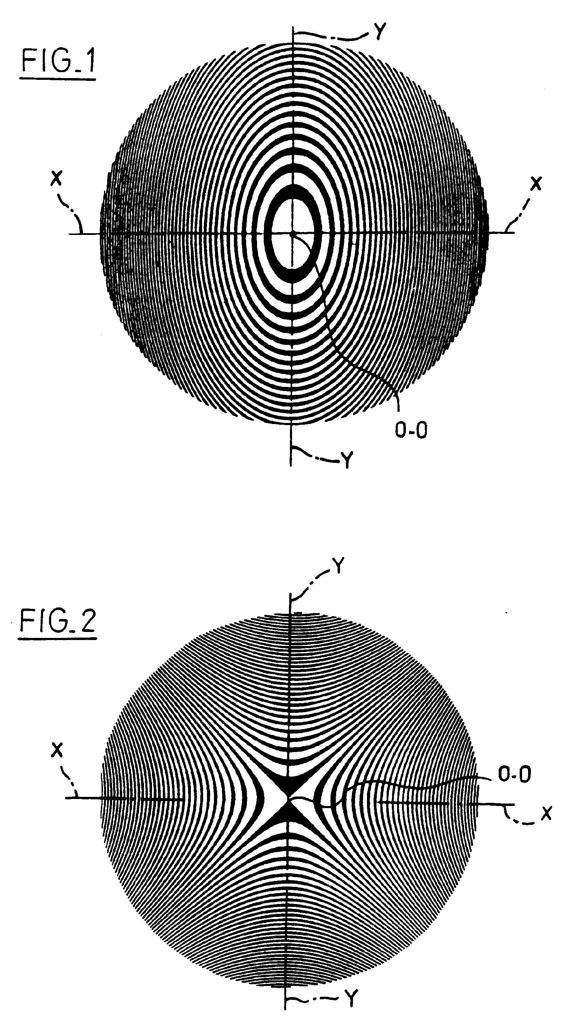

Plus précisément, selon l'invention, les zones diffractives adjacentes sont avantageusement de contour elliptique ou hyperbolique comme illustré respectivement sur les figures 1 et 2.

Ces figures 1 et 2 représentent des vues en plan du contour elliptique ou hyperbolique des composants diffractifs de lentilles conformes à la présente invention. C'est-à-dire que l'axe O-O des lentilles est perpendiculaire au plan des figures 1 et 2.

La représentation donnée sur ces figures est schématique. Sur les figures 1 et 2 les régions alternativement claires et sombres illustrent la périodicité des structures diffractives alternées conformes à l'invention, une zone diffractive dans ce schéma étant constituée d'une région claire, et d'une région foncée adjacente.

Dans la suite de la description, on appellera X et Y deux directions principales, orthogonales entre elles, passant chacune par l'axe O-O de la lentille, et qui coïncident respectivement avec les deux directions orthogonales pour lesquelles l'oeil présente deux focales différentes. Par ailleurs, on appellera $f_x$ et $f_y$ les deux focales de la lentille conforme à la présente invention suivant ces deux directions.

On va dans un premier temps décrire plus en détail les lentilles conformes à la présente invention pourvues de composants diffractifs de contour elliptique du type illustré sur la figure 1.

L'équation de la frontière séparant deux zones diffractives adjacentes de contour elliptique comme illustrées sur la figure 1 est donnée par la relation :

$$x^2/|f_x| + y^2/|f_y| = 2\,\lambda\,k,$$

relation dans laquelle k représente un nombre entier.

L'excentricité des courbes elliptiques ainsi définies dépend de la différence entre les valeurs absolues des focales $f_x$ et $f_y$.

Les composants diffractifs de contour elliptique présentent une double symétrie respectivement par rapport aux directions X et Y qui coïncident avec les axes principaux des courbes elliptiques.

La périodicité en $r^2$, dans la direction X, des composants diffractifs de contour elliptique est : $\Delta r_x^2 = 2\lambda\,|f_x|$.

La périodicité en $r^2$, dans la direction Y, des composants diffractifs de contour elliptique est : $\Delta r_y^2 = 2\lambda\,|f_y|$.

Les composants diffractifs de contour elliptique peuvent avoir un profil de phase du type kinoforme pur,

3

EP 0 382 620 B1

du type kinoforme échantillonné ou encore du type fonction créneau en 0,$\pi$, etc... De plus, le profil de phase $\varphi$ peut être pour chaque zone une fraction croissante ou décroissante de r. De même, la loi de variation (kinoforme pur ou échantillonné, créneau) peut évoluer d'une zone à l'autre.

La valeur absolue de la puissance de correction obtenue dans les directions X et Y dépend de la périodicité respective $\Delta r^2_x$ et $\Delta r^2_y$. La puissance de correction dans la direction X est, en valeur absolue, $2\lambda/\Delta r^2_x$ et dans la direction Y : $2\lambda/\Delta r^2_y$, pour l'ordre +1 ou l'ordre -1 de diffraction.

Le signe de la correction apportée dépend à la fois de l'ordre de diffraction et du signe de d $\varphi$/dr.

Pour les composants diffractifs de contour elliptique ayant un profil de phase du type kinoforme pur ou kinoforme échantillonné, lorsque d$\varphi$/dr est positive, ces composants ne fournissent seulement ou principalement qu'une correction à l'ordre -1 et cette correction est négative, tandis que lorsque d$\varphi$/dr est négative ces composants ne fournissent seulement ou principalement qu'une correction à l'ordre +1 et cette correction est positive.

Pour les composants diffractifs de contour elliptique ayant un profil de phase du type fonction créneau en 0,$\pi$ la correction apportée est positive à l'ordre +1 et négative à l'ordre -1.

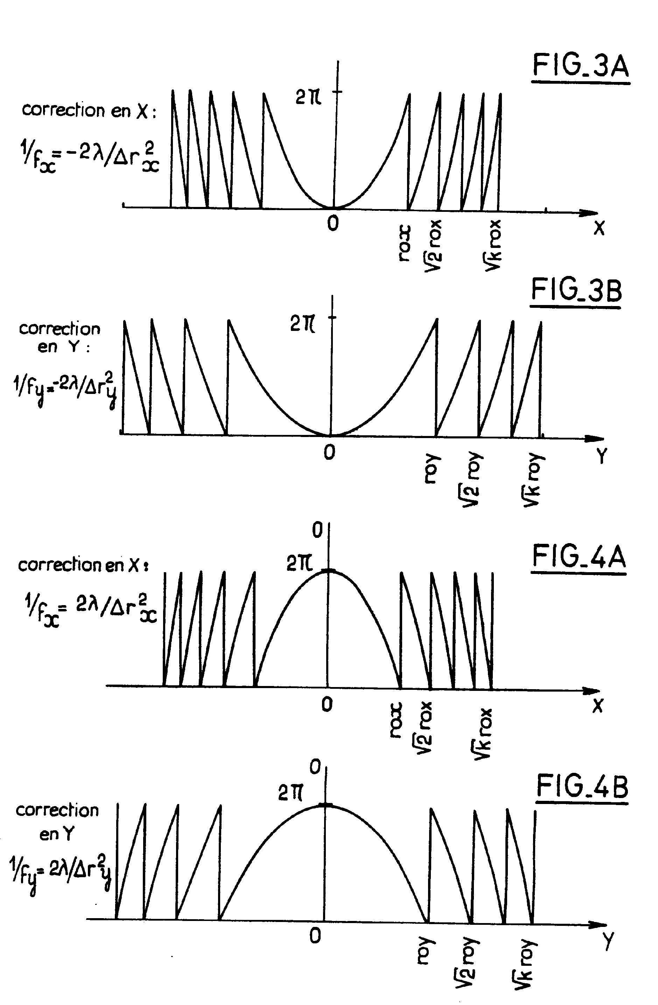

On a représenté sur les figures 3A, 3B ; 4A, 4B ; 5A, 5B ; 6A, 6B ; 7A, 7B ; 8A, 8B ; respectivement deux à deux, les profils de phase en X et Y de 6 composants diffractifs de contour elliptique conformes à la présente invention.

Selon les figures 3A et 3B, le profil de phase des composants diffractifs de contour elliptique est de type kinoforme pur dont la phase varie selon une loi linéaire en $r^2$ de 0 à $2\pi$ en éloignement de l'axe optique O-O. Les composants diffractifs dont les profils de phase sont représentés sur les figures 3A et 3B travaillent dans l'ordre p = -1 seulement à la longueur d'onde d'accord. Ils fournissent respectivement dans les directions X et Y les puissances de correction :

$$+ \ 1/f_x \ = \ - \ 2\lambda \ /\Delta r^2_x$$
$$+ \ 1/f_y \ = \ - \ 2\lambda \ /\Delta r^2_y.$$

Les lentilles équipées de composants diffractifs présentant les profils de phase illustrés sur les figures 3A et 3B corrigent donc une seule amétropie, avec une correction de base de $-2\lambda/\Delta r^2_x$ et $+(2\lambda/\Delta r^2_x - 2\lambda/\Delta r^2_y)$ d'astigmatisme (cylindre d'axe parallèle à X). On notera que cette correction est équivalente à une correction de base de $-2\lambda/\Delta r^2_y$ et $(2\lambda/\Delta r^2_y - 2\lambda/\Delta r^2_x)$ d'astigmatisme (cylindre d'axe parallèle à Y).

Les figures 4A et 4B représentent le profil de phase de composants diffractifs de contour elliptique, également du type kinoforme pur, mais dont la phase varie selon une loi linéaire en $r^2$ de $2\pi$ à 0 en éloignement de l'axe optique O-O (soit de façon inverse aux figures 3A et 3B). Les composants diffractifs dont les profils de phase sont représentés sur les figures 4A et 4B travaillent dans l'ordre p = +1 seulement à la longueur d'onde d'accord. Ils fournissent respectivement dans les directions X et Y les puissances de correction suivantes (de signes inverses de celles indiquées précédemment en regard des figures 3A et 3B) :

$$1/f_x \ = \ 2\lambda/\Delta r^2_x$$
$$1/f_y \ = \ 2\lambda/\Delta r^2_y.$$

Les lentilles équipées de composants diffractifs présentant les profils de phase illustrés sur les figures 4A et 4B corrigent donc une seule amétropie, avec une correction de base de $+2\lambda/\Delta r^2_y$ et $+(2\lambda/\Delta r^2_x - 2\lambda/\Delta r^2_y)$ d'astigmatisme (cylindre d'axe parallèle à Y). On notera que cette correction est équivalente à une correction de base de $+2\lambda/\Delta r_x^2$ et $(2\lambda \ /\Delta r_y^2 - 2\lambda/\Delta r_x^2)$ d'astigmatisme (cylindre d'axe parallèle à X).

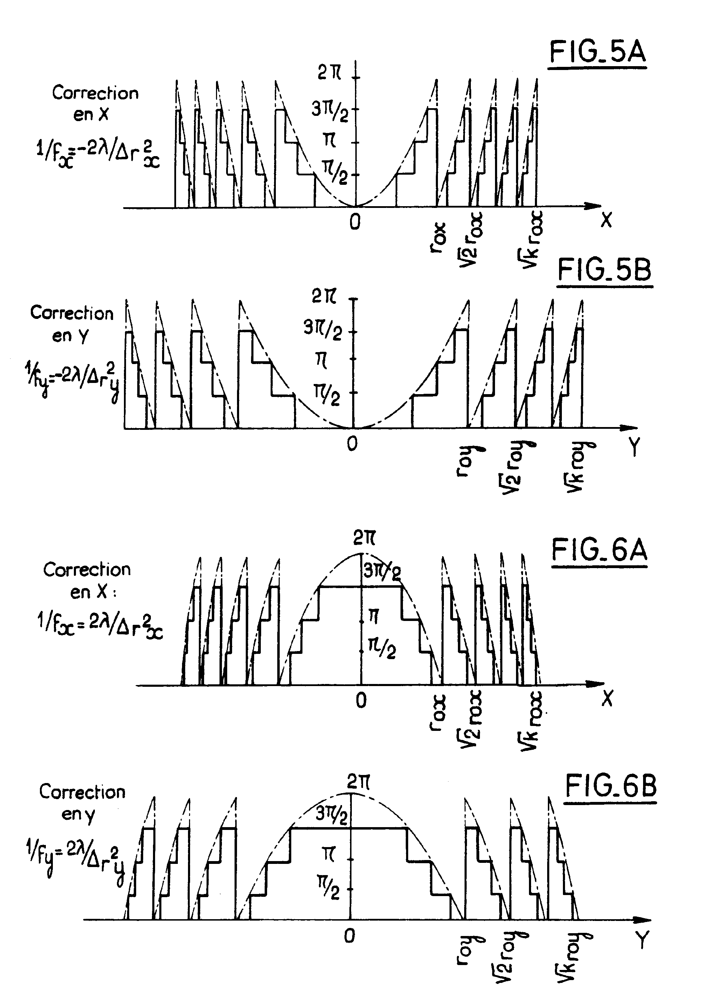

Les figures 5A et 5B représentent le profil de phase de composants diffractifs de contour elliptique du type kinoforme échantillonné en quatre sous-zones déphasées de $2\pi/4$ évoluant de $0,\pi/2,\pi$, 3 $\pi/2$, en éloignement de l'axe optique O-O. Les composants diffractifs dont le profil de phase est représenté sur les figures 5A et 5B travaillent principalement à la longueur d'onde d'accord dans l'ordre p = -1. Ils fournissent dont les mêmes puissances de correction et permettent de corriger la même amétropie que les composants dont le profil de phase est représenté sur les figures 3A et 3B.

Les figures 6A et 6B représentent le profil de phase de composants diffractifs de contour elliptique du type kinoforme échantillonné en 4 sous-zones déphasées de $2\pi/4$ évoluant de $3\pi/2,\pi,\pi/2$ à O en éloignement de l'axe optique O-O. Les composants diffractifs dont les profils de phase sont représentés sur les figures 6A et 6B travaillent principalement à la longueur d'onde d'accord dans l'ordre p = +1. Ils fournissent donc les mêmes puissances de correction et permettent de corriger la même amétropie que les composants dont les profils de phase sont représentés sur les figures 4A et 4B.

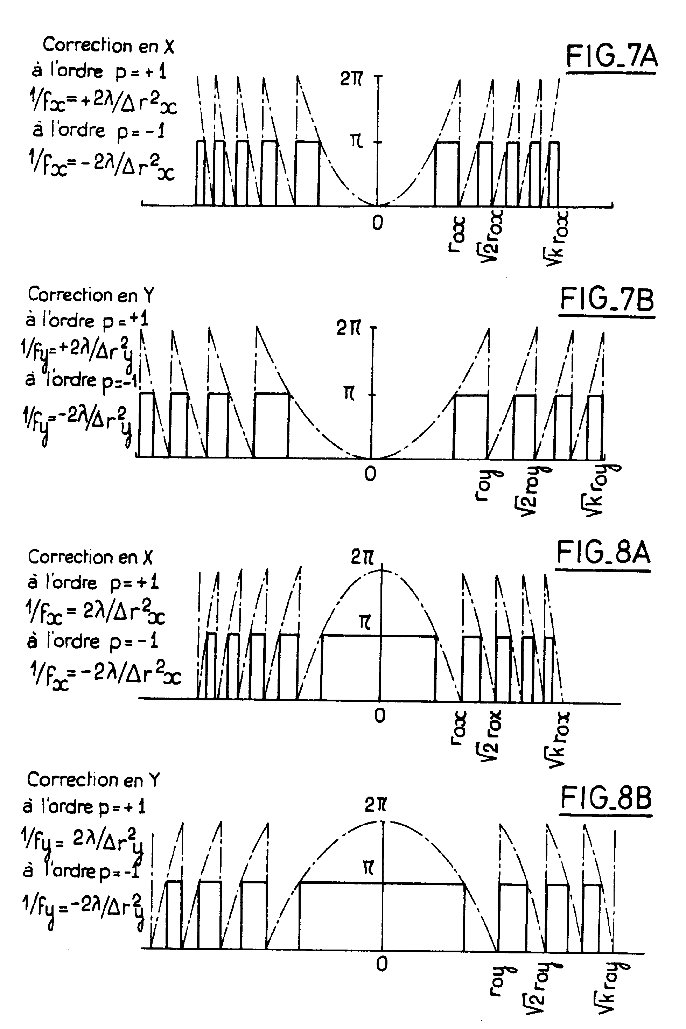

Les figures 7A et 7B représentent le profil de phase de composants diffractifs de contour elliptique de profil binaire comportant deux sous-zones déphasées de $\pi$, plus précisément pour chaque structure diffractive, une zone de déphasage nulle proche de l'axe optique O-O et une zone de déphasage $\pi$ éloignée de l'axe optique O-O. Les composants diffractifs dont les profils de phase sont représentés sur les figures 7A et 7B travaillent à la fois dans l'ordre p = +1 et dans l'ordre p = -1 à la longueur d'onde d'accord, avec une efficacité e = 0,4 dans chaque ordre.

4

A l'ordre p = +1, ils fournissent respectivement dans les directions X et Y les puissances de correction suivantes :

$$+ 1/f_x = + 2\lambda/\Delta r^2_x \text{ et}$$
$$+ 1/f_y = + 2\lambda/\Delta r^2_y.$$

A l'ordre p = -1 les mêmes composants diffractifs fournissent respectivement selon X et Y des puissances de correction de signes opposés, à savoir :

$$1/f_x = - 2\lambda/\Delta r^2_x \text{ et}$$
$$1/f_y = - 2\lambda/\Delta r^2_y.$$

Les lentilles équipées de structures diffractives présentant les profils de phase illustrés sur les figures 7A et 7B peuvent être utilisées pour 4 types de correction.

A l'ordre p = +1 elles fournissent une correction de base de $+2\lambda/\Delta r^2_x$ et $(-2\lambda/\Delta r^2_x + 2\lambda/\Delta r^2_y)$ d'astigmatisme (cylindre d'axe parallèle à X). On notera que cette correction est équivalente à une correction de base de $2\lambda/\Delta r^2_y$ et $(2\lambda/\Delta r^2_x - 2\lambda/\Delta r^2_y)$ d'astigmatisme (cylindre d'axe parallèle à Y).

A l'ordre p = -1 elles fournissent une correction de base de $-2\lambda/\Delta r^2_y$ et $+(2\lambda/\Delta r^2_y - 2\lambda/\Delta r^2_x)$ d'astigmatisme (cylindre d'axe parallèle à Y). On notera que cette correction est équivalente à une correction de base de $-2\lambda/\Delta r^2_x$ et $(2\lambda/\Delta r^2_x - 2\lambda/\Delta r^2_y)$ d'astigmatisme (cylindre d'axe parallèle à X).

Deux autres corrections sont obtenues en combinant les ordres p = +1 et p -1.

A l'ordre p = +1 en X et p = -1 en Y on obtient une correction de base de $2\lambda/\Delta r^2_x$ et $(-2\lambda/\Delta r^2_x - 2\lambda/\Delta r^2_y)$ d'astigmatisme (cylindre d'axe parallèle à X). On notera que cette correction est équivalente à une correction de base de $-2\lambda/\Delta r^2_y$ et $(2\lambda/\Delta r^2_x + 2\lambda/\Delta r^2_y)$ d'astigmatisme (cylindre d'axe parallèle à Y).

A l'ordre p = -1 en X et p = +1 en Y on obtient une correction de base de $-2\lambda/\Delta r^2_x$ et $(2\lambda/\Delta r^2_x + 2\lambda/\Delta r^2_y)$ d'astigmatisme (cylindre d'axe parallèle à X). On notera que cette correction est équivalente à une correction de base de $+2\lambda/\Delta r^2_y$ et $-(2\lambda/\Delta r^2_x + 2\lambda/\Delta r^2_y)$ d'astigmatisme (cylindre d'axe parallèle à Y).

Les figures 8A et 8B représentent le profil de phase de composants diffractifs de contour elliptique, de profil binaire comportant deux sous-zones déphasées de $\pi$, mais possédant une première sous-zone de déphasage $\pi$ proche de l'axe optique O-O et une deuxième sous-zone éloignée de l'axe optique O-O de déphasage nulle.

Les composants diffractifs dont le profil de phase est illustré par les figures 8A et 8B présentent les mêmes puissances de correction aux ordres +1 et -1 que les composants illustrés par les figures 7A et 7B.

Aux lentilles comportant des composants diffractifs tels que décrits ci-avant peut être ajoutée une puissance réfractive additionnelle procurée par la géométrie de la lentille.

Cette puissance réfractive additionnelle est indispensable si l'on veut obtenir une lentille comportant un composant diffractif de structure elliptique, du type kinoforme pur ou échantillonné, lentille dont les puissances de correction sont de signes opposés selon les directions X et Y.

Exemple : soit une lentille comportant un composant diffractif de structure elliptique, du type kinoforme pur ou échantillonné, dont la puissance totale suivant X est <O et la puissance total suivant Y est > O, et dont

$P_x$ diffractive > O, et $P_y$ diffractive > O.

Soit P la puissance réfractive de cette lentille. il est nécessaire que $|P| > P_x$ diffractive pour que

Ptotale suivant X < O,

Ptotale suivant Y > O.

On va maintenant citer trois exemples de correction obtenue à l'aide de composants diffractifs de contour elliptique.

## EXEMPLE 1

Une correction de -4d avec +2d d'astigmatisme (cylindre d'axe parallèle à X) peut être obtenue sans puissance réfractive additionnelle, à l'aide d'une lentille ophtalmique du type illustré sur les figures 3A, 3B ou du type illustré sur les figures 5A, 5B, générant une puissance diffractive $P_x$ = -4d selon l'axe X et une puissance diffractive $P_y$ = -2d selon l'axe Y, soit avec une loi de variation radiale $r_{Kx} = \sqrt{2k\lambda|f_x|} = 0,52 \sqrt{k}$ mm et $rK_y = \sqrt{2k\lambda|f_y|} = 0,74 \sqrt{k}$ mm.

## EXEMPLE 2

Une correction de +2d avec +2d d'astigmatisme (cylindre d'axe parallèle à Y) peut être obtenue sans puissance réfractive additionnelle, à l'aide d'une lentille ophtalmique du type illustré sur les figures 4A, 4B ou du type illustré sur les figures 6A, 6B, générant une puissance diffractive $Px = +4d$ selon l'axe X et une puissance $Py = +2d$ selon l'axe Y, soit avec une loi de variation radiale $rK_x = \sqrt{2k\lambda f_x} = 0{,}52 \sqrt{k}$ mm et $rKy = \sqrt{2k\lambda f_y} = 0{,}74 \sqrt{k}$ mm.

## EXEMPLE 3

Les composants diffractifs dont les profils de phase sont illustrés sur les figures 7A, 7B et 8A, 8B permettant d'obtenir respectivement les mêmes corrections suivant X et Y en prenant la même périodicité radiale $rK_x = \sqrt{2k\lambda f_x} = 0{,}52 \sqrt{k}$ mm et $r_{Ky} = \sqrt{2k\lambda f_y} = 0{,}74 \sqrt{k}$ mm.

En effet les composants répondant aux figures 7A, 7B, et aux figures 8A, 8B à l'ordre $p = -1$ fournissent alors une correction de base de -4d avec +2d d'astigmatisme (cylindre d'axe parallèle à X). Par ailleurs, les mêmes composants répondant aux figures 7A, 7B, et aux figures 8A, 8B, mais à l'ordre $p = +1$, fournissant une correction de base de +2d avec +2d d'astigmatisme (cylindre d'axe parallèle à Y).

On va dans un second temps décrire plus en détail les lentilles optiques conformes à la présente invention pourvues de composants diffractifs de contour hyperbolique du type illustré sur la figure 2.

Les frontières séparant les zones diffractives adjacentes de contour hyperbolique sont en fait définies par deux équations :

$$x^2/\left|f_x\right| - y^2/\left|f_y\right| = 2k\lambda$$

et

$$y^2/\left|f_y\right| - x^2/\left|f_x\right| = 2k\lambda ,$$

dans lesquelles k représente un nombre entier.

Les lentilles optiques pourvues de composants diffractifs de contour hyperbolique comprennent en effet deux séries de composants diffractifs sont les frontières sont définies par des courbes hyperboliques dont les axes focaux coïncident respectivement avec les axes X et Y.

Les composants diffractifs de contour hyperbolique présentent donc également une double symétrie respectivement par rapport aux axes X et Y qui coïncident avec les axes focaux des courbes hyperboliques.

La périodicité en $r^2$, suivant l'axe X, des composants diffractifs de contour hyperbolique est : $\Delta r^2{}_x = \left| 2\lambda f_x \right|$.

La périodicité en $r^2$, suivant l'axe Y, des composants diffractifs de contour hyperbolique est : $\Delta r^2{}_y = \left| 2\lambda f_y \right|$.

Les composants diffractifs de contour hyperbolique peuvent avoir un profil de phase du type kinoforme pur, du type kinoforme échantillonné ou encore du type fonction créneau en $0,\pi$.

De plus, le profil de phase $\varphi$ peut être pour chaque zone une fonction croissante ou décroissante de r. De même, la loi de variation (kinoforme ou échantillonné, créneau... ) peut évoluer d'une zone à l'autre. Comme les corrections $f_x$ et $f_y$ sont de signes opposés pour un composant diffractif de type kinoforme pur ou échantillonné, les sens de variation du profil de phase $\varphi$ selon l'axe X et selon l'axe Y sont donc de signes inverses. $d\varphi/drx$ et $d\varphi/dry$ sont donc de signes opposés, ce qui est illustré sur la figure 2 par la discontinuité au niveau des asymptotes.

La valeur absolue de la puissance de correction obtenue dans les plans X et Y dépend de la périodicité respective $\Delta r^2{}_x$ et $\Delta r^2{}_y$. La puissance de correction selon l'axe X est, en valeur absolue, $2\lambda/\Delta r^2{}_x$ et selon l'axe Y : $2\lambda/\Delta r^2{}_y$.

Le signe de la correction apportée dépend à la fois de l'ordre de diffraction et du sens (croissant ou décroissant en éloignement de l'axe O-O de la lentille) d'évolution du profil de phase.

Pour les composants diffractifs de contour hyperbolique ayant un profil de phase du type kinoforme pur ou kinoforme échantillonné, la correction apportée est négative lorsque dans la direction considérée $d\varphi/dr$ est positive et la correction apportée est positive lorsque dans la direction considérée $d\varphi/dr$ est négative.

Pour les composants diffractifs de contour hyperbolique ayant un profil de phase du type fonction créneau en $0,\pi$ la correction apportée dans une direction est de même signe que celui de l'ordre de diffraction considéré.

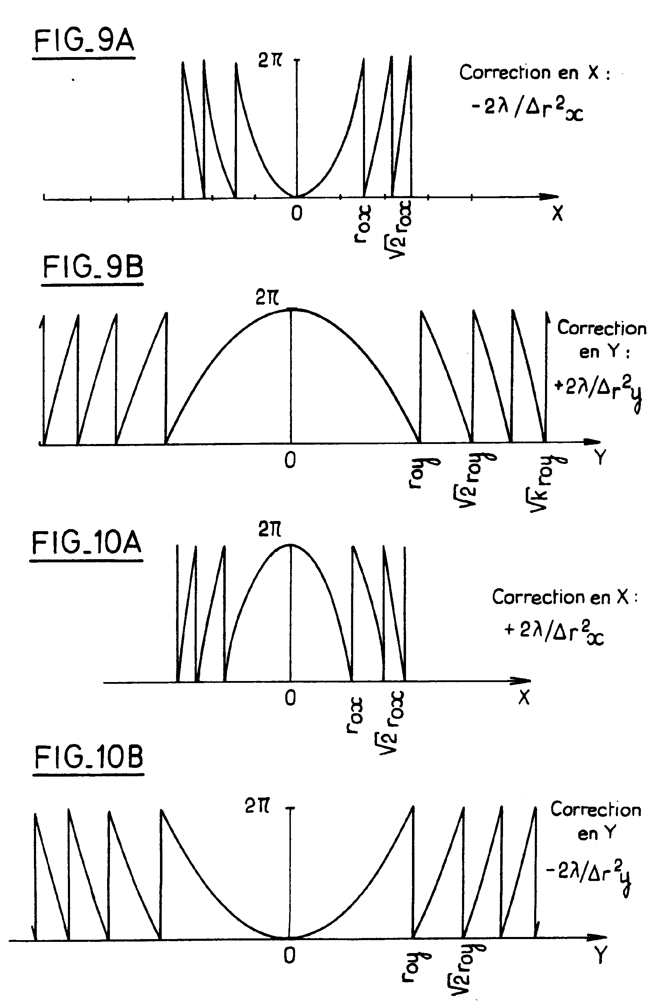

On a représenté sur les figures 9A, 9B ; 10A, 10B ; 11A, 11B ; 12A, 12B ; 13A, 13B ; 14A, 14B ; 15A, 15B ; respectivement deux à deux les profils de phase en X et Y de sept composants diffractifs de contour hyperbolique conformes à la présente invention.

Selon les figures 9A, 9B, le profil de phase des composants diffractifs de contour hyperbolique est de type kinoforme pur dont la phase varie selon une loi linéaire en $r^2$ de 0 à $2\pi$ en éloignement de l'axe optique O-O selon l'axe X et selon une loi linéaire en $r^2$ de $2\pi$ à 0 en éloignement de l'axe optique O-O, selon l'axe Y. Ces composants travaillant dans l'ordre $p = -1$ en X et $p = +1$ en Y seulement à la longueur d'onde d'accord. Ils fournissent respectivement en X et Y les puissances de correction :

$$+ 1/f_x = - 2\lambda/\Delta r^2{}_x$$

$$+ \; 1/f_y \; = \; + \; 2\lambda/\Delta r^2_y.$$

Les lentilles équipées de composants diffractifs présentant les profils de phase illustrés sur les figures 9A et 9B corrigent donc une seule amétropie avec une correction de base de - $2\lambda/\Delta r^2_x$ et + ($2\lambda/\Delta r^2_x + 2\lambda/\Delta r^2_y$) d'astigmatisme (cylindre d'axe parallèle à X). On notera que cette correction est équivalente a une correction de base +$2\lambda/\Delta r^2_y$ et -($2\lambda/\Delta r^2_x + 2\lambda/\Delta r^2_y$) d'astigmatisme (cylindre d'axe parallèle à Y).

Selon les figures 10A, 10B, le profil de phase des composants diffractifs de contour hyperbolique est de type kinoforme pur dont la phase varie selon une loi linéaire en $r^2$ de $2\pi$ à 0 en éloignement de l'axe optique O-O selon l'axe X et selon une loi linéaire en $r^2$ de 0 à $2\pi$ en éloignement de l'axe optique O-O, selon l'axe Y. Ces composants travaillent dans l'ordre p = + 1 en X et p = -1 en Y seulement à la longueur d'onde d'accord. Ils fournissent respectivement selon X et Y les puissances de correction :

$$+ \; 1/f_x \; = \; + \; 2\lambda/\Delta r^2_x$$
$$+ \; 1/f_y \; = \; - \; 2\lambda/\Delta r^2_y.$$

Les lentilles équipées de composants diffractifs présentant les profils de phase illustrés sur les figures 10A et 10B corrigent donc une seule amétropie avec une correction de base de - $2\lambda/\Delta r^2_y$ et + ($2\lambda/\Delta r^2_x + 2\lambda/\Delta r^2_y$) d'astigmatisme (cylindre d'axe parallèle à Y). On notera que cette correction est équivalente à une correction de base de +$2\lambda/\Delta r^2_x$ et -($2\lambda/\Delta r^2_x + 2\lambda/\Delta r^2_y$) d'astigmatisme (cylindre d'axe parallèle à Y).

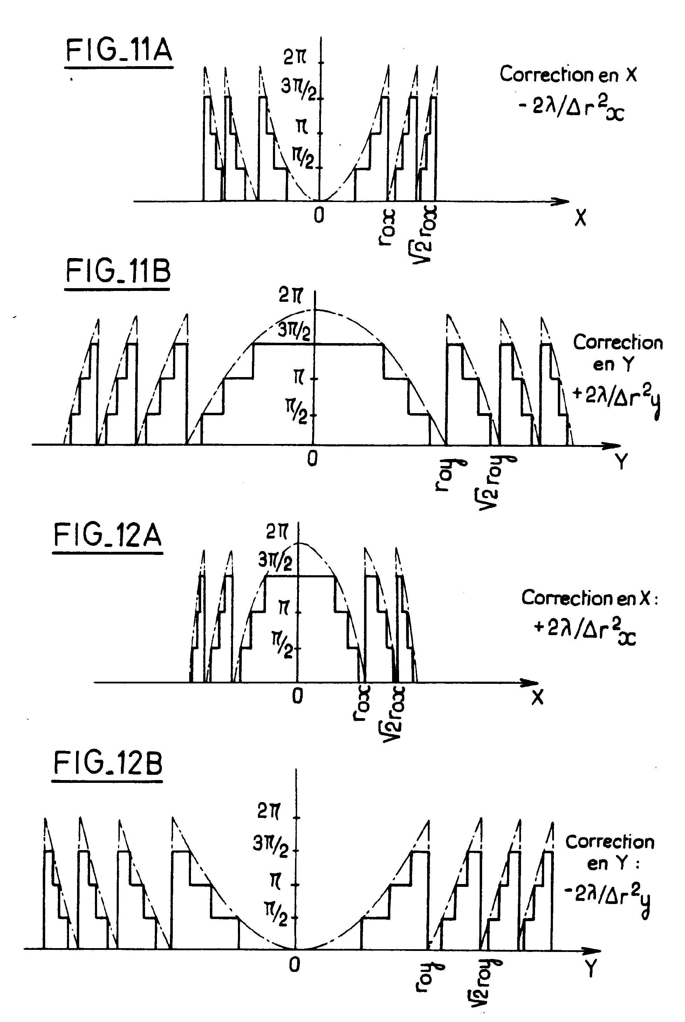

Selon les figures 11A, 11B, le profil de phase des composants diffractifs de contour hyperbolique est de type kinoforme échantillonné en quatre sous zones déphasées de $2\pi/4$ dont la phase varie de façon croissante de 0 à $\pi/2$, $\pi$, $3\pi/2$ en éloignement de l'axe optique O-O selon l'axe X et varie de façon décroissante de $3\pi/2$ à $\pi$, $\pi/2$ et 0 en éloignement de l'axe optique O-O, selon l'axe Y. Ces composants travaillant principalement dans l'ordre p = -1 en X et p = +1 en Y à la longueur d'onde d'accord. Ils fournissent respectivement selon X et Y les mêmes puissances de correction que dans le cas des figures 9A, 9B :

$$+ \; 1/f_x \; = \; - \; 2\lambda/\Delta r^2_x$$
$$+ \; 1/f_y \; = \; + \; 2\lambda/\Delta r^2_y.$$

Les lentilles équipées de composants diffractifs présentant les profils de phase illustrés sur les figures 11A et 11B corrigent donc la même amétropie que les lentilles illustrées sur les figures 9A et 9B.

Selon les figures 12A, 12B, le profil de phase des composants diffractifs de contour hyperbolique est de type kinoforme échantillonné en quatre sous zones déphasées de $2\pi/4$ dont la phase varie de façon décroissante de 3 $\pi/2$, $\pi$,$\pi/2$, à 0 en éloignement de l'axe optique O-O selon l'axe X et varie de façon croissante de 0 à $\pi/2$,$\pi$, $3\pi/2$ en éloignement de l'axe optique O-O, selon l'axe Y. Ces composants travaillent principalement dans l'ordre p = + 1 en X et p = -1 en Y à la longueur d'onde d'accord. Ils fournissent respectivement selon X et Y les mêmes puissances de correction que dans le cas des figures 10A, 10B :

$$+ \; 1/f_x \; = \; + \; 2\lambda/\Delta r^2_x$$
$$+ \; 1/f_y \; = \; - \; 2\lambda/\Delta r^2_y.$$

Les lentilles équipées de composants diffractifs présentant les profils de phase illustrés sur les figures 12A et 12B corrigent donc la même amétropie que les lentilles illustrées sur les figures 10A et 10B.

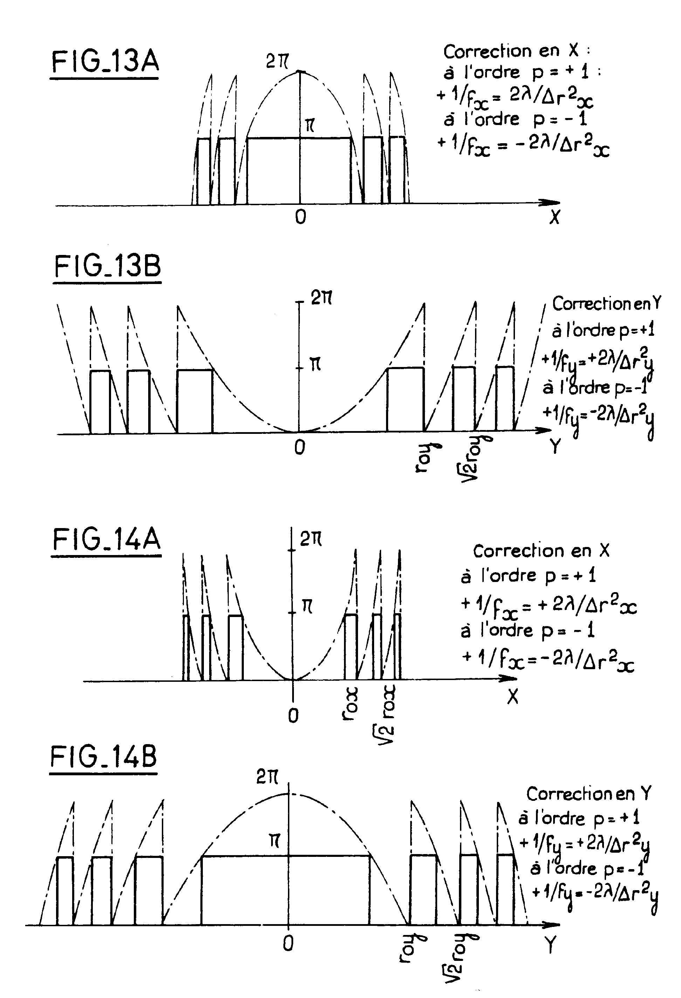

Selon les figures 13A, 13B, le profil de phase des composants diffractifs de contour hyperbolique est de type binaire comportant deux sous zones déphasées de $\pi$, plus précisément, pour chaque structure diffractive, selon l'axe X une zone de déphasage $\pi$ proche de l'axe optique O-O de la lentille et une zone de déphasage nul éloignée de l'axe optique O-O et selon l'axe Y une zone de déphasage nul proche de l'axe optique O-O de la lentille et une zone de déphasage $\pi$ éloignée de l'axe optique O-O. Ces composants travaillent simultanément à l'ordre p = + 1 et à l'ordre p = - 1.

A l'ordre p = + 1, les composants diffractifs fournissent respectivement selon les directions X et Y les puissances de correction :

$$+ \; 1/f_x \; = \; + \; 2\lambda/\Delta r^2_x \; \text{et}$$
$$+ \; 1/f_y \; = \; + \; 2\lambda/\Delta r^2_y.$$

A l'ordre p = - 1 les composants diffractifs fournissent respectivement selon les directions X et Y les puissances de correction :

$$+ \; 1/f_x \; = \; - \; 2\lambda/\Delta r^2_x \; \text{et}$$
$$+ \; 1/f_y \; = \; - \; 2\lambda/\Delta r^2_y.$$

Les lentilles équipées de composants diffractifs présentant les profils de phase illustrés sur les figures 13A et 13B peuvent être utilisées pour 4 types de corrections, qui correspond à celles des lentilles représentées sur les figures 7A, 7B et évoquées ci-dessus.

Selon les figures 14A, 14B, le profil de phase des composants diffractifs de contour hyperbolique est de type binaire comportant deux sous zones déphasées de $\pi$, plus précisément, pour chaque structure diffractive, selon l'axe X une zone de déphasage nul proche de l'axe optique O-O de la lentille et une zone de déphasage $\pi$ éloignée de l'axe optique O-O et selon l'axe Y une zone de déphasage $\pi$ proche de l'axe optique O-O de la lentille et une zone de déphasage nul éloignée de l'axe optique O-O.

Ces composants présentent exactement les mêmes puissances de correction aux ordres +1 et -1 que les composants illustrés par les figures 13A et 13B.

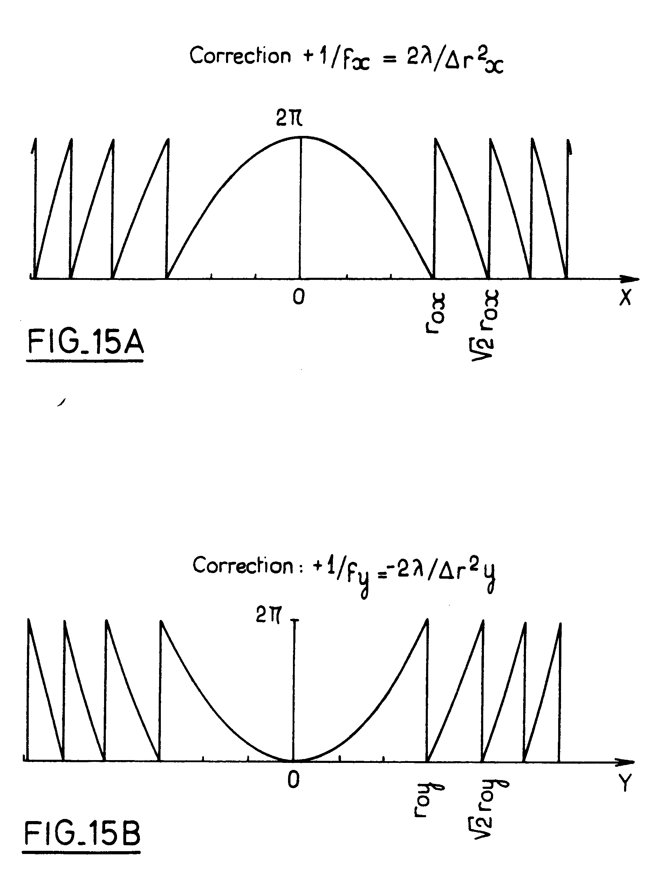

On a représenté sur les figures 15A, 15B le profil de phase d'un composant hyperbolique particulier dont la périodicité est identique selon les axes X et Y.

Quel que soit le profil de phase retenu (il est de type kinoforme pur selon les figures 15A, 15B, mais pourrait également être du type kinoforme échantillonné ou du type profil binaire en fonction créneau $0,\pi$), pour un réseau d'hyperboles équilatères, les focales suivant X et Y ont toujours même valeur absolue.

Bien entendu on peut associer, à une lentille diffractive comportant un composant diffractif de type hyperbolique, une puissance réfractive additionnelle procurée par la géométrie de la lentille afin d'amener les deux focales $f_x$ et $f_y$ dans le domaine de correction souhaité.

On va maintenant citer différents exemples de correction obtenue à l'aide de composants diffractifs de contour hyperbolique.

EXEMPLE 4

Une correction de - 2d avec + 3d d'astigmatisme (cylindre d'axe X) peut être obtenue sans puissance réfractive additionnelle à l'aide d'une lentille du type illustré sur les figures 9A, 9B ou du type illustré sur les figures 11A, 11B, générant une puissance diffractive Px = -2d selon l'axe X et une puissance diffractive Py = + 1d selon l'axe Y, soit avec une loi de variation radiale $rK_x = \sqrt{|2\,k\lambda f_x|} = 0,74\ \sqrt{k}$ mm et $rK_y = \sqrt{|2\,k\lambda f_y|} = 1,04\ \sqrt{k}$ mm.

EXEMPLE 5

Une correction de - 1d avec + 3d d'astigmatisme (cylindre d'axe Y) peut être obtenue sans puissance réfractive additionnelle à l'aide d'une lentille du type illustré sur les figures 10A, 10B, ou du type illustré sur les figures 12A, 12B, générant une puissance diffractive Px = + 2d selon l'axe X et une puissance diffractive Py = - 1d selon l'axe Y, soit selon une loi de variation radiale $rK_x = \sqrt{|2\,k\lambda f_x|} = 0,74\ \sqrt{k}$ mm et $r_{Ky} = \sqrt{|2\,k\lambda f_y|} = 1,04\ \sqrt{k}$ mm.

EXEMPLE 6

Les composants diffractifs dont les profils de phase sont illustrés sur les figures 13A, 13B, et 14A, 14B présentent exactement les mêmes puissances de correction et peuvent être utilisés pour les deux types de correction cités ci-dessus dans les exemples 4 et 5 en prenant la même loi de variation radiale $rK_x = \sqrt{|2\,k\lambda f_x|} = 0,74\ \sqrt{k}$ mm et $r_{Ky} = \sqrt{|2\,k\lambda f_y|} = 1,04\ \sqrt{k}$ mm.

En effet, les composants répondant aux figures 13A, 13B, et aux figures 14A, 14B, à l'ordre p = - 1 en X et à l'ordre p = +1 en Y fournissent alors une correction de base de - 2d avec + 3d d'astigmatisme (cylindre d'axe à X).

Par ailleurs, les mêmes composants répondant aux figures 13A, 13B, et aux figures 14A, 14B, mais à l'ordre p = + 1 en X et à l'ordre p = -1 en Y, fournissent alors une correction de base de - 1d avec + 3d d'astigmatisme (cylindre d'axe Y).

Il est essentiel de noter qu'avec un composant de contour hyperbolique de type kinoforme pur ou échantillonné les puissances selon les axes X et Y sont toujours de signe opposé.

Par conséquent, il est impossible d'obtenir uniquement au moyen d'un composant diffractif de contour hyperbolique de type kinoforme pur ou échantillonnédes lentilles dont les puissances selon X et Y sont de même signe.

Si les puissances de la lentille sont de même signe, il est nécessaire de doter la lentille, à laquelle est associée ce composant, d'une puissance réfractive, comme illustré par l'exemple 7 ci-après.

EXEMPLE 7

Une correction de - 4d avec + 2d d'astigmatisme (cylindre d'axe Y) peut être obtenue avec une puissance réfractive additionnelle de - 3d, à l'aide d'une lentille de contact du type hyperbole avec $\Delta r^2_x = \Delta r^2_y$ à profil kinoforme pur du type illustré sur les figures 15A et 15B générant une puissance diffractive Px = + 1d selon l'axe X et une puissance diffractive Py = - 1d selon l'axe Y, soit $rK_x = \sqrt{|2\,k\lambda f_x|} = 1,04\ \sqrt{k}$ mm et $rK_y =$

$\sqrt{|2\,k\lambda f_y|} = 1,04\,\sqrt{k}$ mm.

Les composants diffractifs conformes à la présente invention sont de préférence formés par variation d'indice, bien que en théorie ces composants puissent également être réalisés par exemple par relief.

On notera en outre qu'il est nécessaire d'orienter le composant diffractif par rapport aux axes horizontal et vertical de la lentille.

L'astigmatisme que peut présenter l'oeil d'un individu est en effet rarement orienté à 0° (axe horizontal) ou à 90° (axe vertical) mais est généralement distinct de ces deux axes de référence.

Par ailleurs, une lentille de contact pour la correction de l'astigmatisme doit avoir une orientation angulaire sur l'oeil parfaitement défini et stable, c'est-à-dire n'être l'objet d'aucune rotation sur elle-même dûe par exemple aux mouvements des paupières, et essentiellement de la paupière supérieure.

Pour cette raison les lentilles de contact pour astigmates comporteront selon l'invention des moyens permettant de les stabiliser en orientation sur l'oeil. Ces moyens pourront être choisis dans le groupe comprenant :

- un prisme ballast tel qu'enseigné dans le document EP-A-0 008 726,
- une troncature horizontale à la partie inférieure de la lentille,
- des zones supérieure et inférieure allégées (amincies),
- une zone externe torique telle qu'enseignée dans le document FR-A-2 425 088, ou encore
- un ou des bossage(s) placé(s) sur l'axe de stabilisation horizontale, tel(s) qu'enseigné(s) dans le document EP-A 0 042 023.

Bien entendu la présente invention n'est pas limitée aux modes de réalisation particuliers qui viennent d'être décrits mais s'étend à toutes variantes conformes à son esprit.

## Revendications

1.  Lentille diffractive ophtalmique pour la correction de l'astigmatisme, caractérisée par le fait qu'elle comprend des composants diffractifs dont le contour est délimité par des courbes coniques à centre non dégénérées, du type hyperbole ou ellipse, à l'exclusion des cercles.

2.  Lentille selon la revendication 1, caractérisée par le fait qu'elle comprend des composants diffractifs adjacents de contour hyperbolique ou elliptique dont la périodicité en $r^2$ selon deux directions X, Y orthogonales entre elles, passant par l'axe de la lentille et coïncidant avec les axes principaux $r_x$, $r_y$ des hyperboles ou ellipses est déterminée respectivement par les relations :
$$\Delta r_x{}^2 = 2\lambda\,|f_x|\,,$$
$$\Delta r_y{}^2 = 2\lambda\,|f_y|$$
dans lesquelles :
$\Delta r_x{}^2$      représente la périodicité en $r^2$ selon X,
$\Delta r_y{}^2$      représente la périodicité en $r^2$ selon Y,
$\lambda$      représente la longueur d'onde moyenne d'utilisation,
$f_x$      représente la focale recherchée dans la direction X, et
$f_y$      représente la focale recherchée dans la direction Y.

3.  Lentille selon l'une des revendications 1 ou 2, caractérisée par le fait que les composants diffractifs sont de contour elliptique défini par l'équation :
$$x^2/|f_x| + y^2/|f_y| = 2\lambda\,k,$$
relation dans laquelle k représente un nombre entier.

4.  Lentille selon l'une des revendications 1 et 2, caractérisée par le fait que les composants diffractifs sont de contour hyperbolique défini par les équations :
$$x^2/|f_x| - y^2/|f_y| = 2\lambda\,k$$
et
$$y^2/|f_y| - x^2/|f_x| = 2\,\lambda\,k$$
dans lesquelles
k      représente un nombre entier.

5.  Lentille selon la revendication 1 à 4, caractérisée par le fait que les composants diffractifs sont formés par variation d'indice.

6. Lentille selon l'une des revendications 1 à 5 formant lentille de contact et destinée à être placée sur la cornée de l'oeil, caractérisée par le fait qu'elle comprend des moyens de stabilisation en orientation.

7. Lentille selon la revendication 6, caractérisée par le fait que les moyens de stabilisation sont choisis dans le groupe comprenant : un prisme ballast, une troncature horizontale à la partie inférieure de la lentille, des zones supérieure et inférieure allégées, une zone externe torique et/ou un bossage placé sur l'axe de stabilisation horizontale.

8. Lentille selon l'une des revendications 1 à 7, caractérisée par le fait que les composants diffractifs ont un profil de phase du type kinoforme pur évoluant entre 0 et $2\pi$.

9. Lentille selon l'une des revendications 1 à 7, caractérisée par le fait que les composants diffractifs ont un profil de phase du type kinoforme échantillonné en n sous zones déphasées de $2\pi/n$.

10. Lentille selon l'une des revendications 1 à 7, caractérisée par le fait que les composants diffractifs ont un profil de phase binaire comportant deux sous zones déphasées de $\pi$.

**Patentansprüche**

1. Diffraktive ophtalmische Linse zur Astigmatismuskorrektur, dadurch gekennzeichnet, daß sie diffraktive Bestandteile umfaßt, deren Kontur durch konische, nicht entartete Mittelpunktkurven des hyperbolischen oder elliptischen Typs unter Ausschluß von Kreisen begrenzt ist.

2. Linse nach Anspruch 1, dadurch gekennzeichnet, daß sie diffraktive Bestandteile an der hyperbolischen oder elliptischen Kontur umfaßt, deren Periodizität mit $r^2$ längs der orthogonalen Richtungen X, Y, die durch die Achse der Linse verlaufen und mit den Hauptachsen $r_x$, $r_y$ der Hyperbeln oder Ellipsen zusammenfallen, jeweils durch die Beziehungen bestimmt ist:
$$\Delta r_x{}^2 = 2\lambda \, |f_x|,$$
$$\Delta r_y{}^2 = 2\lambda \, |f_y|$$
wobei:
$\Delta r_x{}^2$ die Periodizität mit $r^2$ längs X darstellt,
$\Delta r_y{}^2$ die Periodizität mit $r^2$ längs Y darstellt,
$\lambda$ die mittlere verwendete Wellenlänge darstellt,
$f_x$ den Fokus in Richtung X und
$f_y$ den Fokus in Richtung Y darstellt.

3. Linse nach einem der Ansprüche 1 oder 2, dadurch gekennzeichnet, daß die diffraktiven Bestandteile eine elliptische Kontur haben, die durch die Gleichung bestimmt ist:
$$x^2/|f_x| + y^2/|f_y| = 2\lambda k,$$
wobei k eine ganze Zahl ist.

4. Linse nach einem der Ansprüche 1 und 2, dadurch gekennzeichnet, daß die diffraktiven Bestandteile eine hyperbolische Kontur haben, die durch die Gleichungen bestimmt sind:
$$x^2/|f_x| - y^2/|f_y| = 2\lambda k$$
und
$$y^2/|f_y| - x^2/|f_x| = 2\lambda k,$$
wobei k eine ganze Zahl ist.

5. Linse nach Anspruch 1 bis 4, dadurch gekennzeichnet, daß die diffraktiven Bestandteile durch eine Variation des Brechungsindexes gebildet sind.

6. Linse nach einem der Ansprüche 1 bis 5, welche aus einer Kontaktlinse besteht und zum Aufsetzen auf die Hornhaut des Auges vorgesehen ist, dadurch gekennzeichnet, daß sie Mittel zur Stabilisierung der Richtung umfaßt.

7. Linse nach Anspruch 6, dadurch gekennzeichnet, daß die Stabilisierungsmittel gewählt sind aus der Gruppe bestehend aus einem Balastprisma, einer horizontalen Verkürzung im unteren Teil der Linse, verdünnten oberen und unteren Zonen, einer äußeren torischen Zone und/oder einem Vorsprung, der auf der horizontalen Stabilisierungsachse angeordnet ist.

8. Linse nach einem der Ansprüche 1 bis 7, dadurch gekennzeichnet, daß die diffraktiven Bestandteile ein Phasenprofil des reinen kinoformen Typs zwischen 0 und $2\pi$ haben.

9. Linse nach einem der Ansprüche 1 bis 7, dadurch gekennzeichnet, daß die diffraktiven Bestandteile ein Phasenprofil des gemusterten, kinoformen Typs mit n Teilzonen haben, die um $2\pi/n$ phasenverschoben sind.

10. Linse nach einem der Ansprüche 1 bis 7, dadurch gekennzeichent, daß die diffraktiven Bestandteile ein binäres Phasenprofil haben, das zwei um $\pi$ phasenverschobene Teilzonen umfaßt.

## Claims

1. A diffractive eye lens for correcting astigmatism, characterized by the fact that it includes diffractive components whose outlines are delimited by conic section curves having non-degenerative centers of the type hyperbolic or elliptical, with the exclusion of the circles.

2. A lens according to claim 1, characterized by the fact that it includes adjacent hyperbolic- or elliptical-outline diffractive components whose periodicity in $r^2$ in two mutually orthogonal X and Y directions intersecting at the axis of the lens and coinciding with the main axes rx. ry of the hyperbolas or the ellipses are respectively determined by the equations:
$$\Delta r_x^2 = 2\lambda \left| f_x \right|;$$
and
$$\Delta r_y^2 = 2\lambda \left| f_y \right|;$$
in which:

$\Delta r_x^2$      represents the periodicity in $r^2$ along the X direction;

$\Delta r_y^2$      represents the periodicity in $r^2$ along the Y direction;

$\lambda$      represents the mean utilization wavelength;

$f_x$      represents the desired focal length in the X direction; and

$f_y$      represents the desired focal length in the Y direction.

3. A lens according to claim 1 or 2, characterized by the fact that the diffractive components have elliptical outlines defined by the equation:
$$x^2/\left| f_x \right| + y^2/\left| f_y \right| = 2\lambda k$$
where $\underline{k}$ represents an integer.

4. A lens according to claim 1 or 2, characterized by the fact that the diffractive components have hyperbolic outlines defined by the equations:
$$x^2/\left| f_x \right| - y^2/\left| f_y \right| = 2\lambda k;$$
and
$$y^2/\left| f_y \right| - x^2/\left| f_x \right| = 2\lambda k$$
in which $\underline{k}$ represents an integer.

5. A lens according to any one of claims 1 to 4, characterized by the fact that the diffractive components are made by index variation.

6. A lens according to any one of claims 1 to 5, constituting a contact lens intended to be placed on the cornea of the eye, characterized by the fact that it includes orientation stabilizing means.

7. A lens according to claim 6, characterized by the fact that the stabilizing means are selected from the group comprising: a ballasting prism; horizontal truncation at the bottom of the lens; top and bottom zones of reduced weight; an outer toroidal zone; and a bulge placed on the horizontal stabilization axis.

8. A lens according to any one of claims 1 to 7, characterized by the fact that the diffractive components have a pure kinoform type phase profile varying between 0 and $2\pi$.

9. A lens according to any one of claims 1 to 7; characterized by the fact that the diffractive components have a sampled kinoform type phase profile comprising <u>n</u> subzones at phase differences of $2\pi/n$.

10. A lens according to any one of claims 1 to 7, characterized by the fact that the diffractive components have a binary phase profile comprising two subzones at a phase difference of $\pi$.

FIG.1

FIG.2

EP 0 382 620 B1

FIG. 3A

correction en X :

$$\frac{1}{f_x} = -2\lambda/\Delta r_x^2$$

FIG. 3B

correction en Y :

$$\frac{1}{f_y} = -2\lambda/\Delta r_y^2$$

FIG. 4A

correction en X :

$$\frac{1}{f_x} = 2\lambda/\Delta r_x^2$$

FIG. 4B

correction en Y

$$\frac{1}{f_y} = 2\lambda/\Delta r_y^2$$

14

FIG.5A

Correction
en X

$1/f_x = -2\lambda/\Delta r_x^2$

$2\pi$
$3\pi/2$
$\pi$
$\pi/2$

$0$

$r_{ox}$  $\sqrt{2}\,r_{ox}$  $\sqrt{k}\,r_{ox}$  X

FIG.5B

Correction
en Y

$1/f_y = -2\lambda/\Delta r_y^2$

$2\pi$
$3\pi/2$
$\pi$
$\pi/2$

$0$

$r_{oy}$  $\sqrt{2}\,r_{oy}$  $\sqrt{k}\,r_{oy}$  Y

FIG.6A

Correction
en X :

$1/f_x = 2\lambda/\Delta r_x^2$

$2\pi$
$3\pi/2$
$\pi$
$\pi/2$

$0$

$r_{ox}$  $\sqrt{2}\,r_{ox}$  $\sqrt{k}\,r_{ox}$  X

FIG.6B

Correction
en y

$1/f_y = 2\lambda/\Delta r_y^2$

$2\pi$
$3\pi/2$
$\pi$
$\pi/2$

$0$

$r_{oy}$  $\sqrt{2}\,r_{oy}$  $\sqrt{k}\,r_{oy}$  Y

Correction en X
à l'ordre $p = +1$
$1/f_{x} = +2\lambda/\Delta r^2_{x}$
à l'ordre $p = -1$
$1/f_{x} = -2\lambda/\Delta r^2_{x}$

FIG_7A

$2\pi$

$\pi$

$0$

$r_{ox}$  $\sqrt{2} r_{ox}$  $\sqrt{k} r_{ox}$

Correction en Y
à l'ordre $p = +1$
$1/f_{y} = +2\lambda/\Delta r^2_{y}$
à l'ordre $p = -1$
$1/f_{y} = -2\lambda/\Delta r^2_{y}$

FIG_7B

$2\pi$

$\pi$

$0$

$r_{oy}$  $\sqrt{2} r_{oy}$  $\sqrt{k} r_{oy}$

Correction en X
à l'ordre $p = +1$
$1/f_{x} = 2\lambda/\Delta r^2_{x}$
à l'ordre $p = -1$
$1/f_{x} = -2\lambda/\Delta r^2_{x}$

FIG_8A

$2\pi$

$\pi$

$0$

$r_{ox}$  $\sqrt{2} r_{ox}$  $\sqrt{k} r_{ox}$

Correction en Y
à l'ordre $p = +1$
$1/f_{y} = 2\lambda/\Delta r^2_{y}$
à l'ordre $p = -1$
$1/f_{y} = -2\lambda/\Delta r^2_{y}$

FIG_8B

$2\pi$

$\pi$

$0$

$r_{oy}$  $\sqrt{2} r_{oy}$  $\sqrt{k} r_{oy}$

16

## FIG_9A

$2\pi$

Correction en X :

$-2\lambda/\Delta r^2_x$

0    $r_{ox}$    $\sqrt{2}r_{ox}$    X

## FIG_9B

$2\pi$

Correction
en Y :

$+2\lambda/\Delta r^2_y$

0    $r_{oy}$    $\sqrt{2}r_{oy}$    $\sqrt{k}r_{oy}$    Y

## FIG_10A

$2\pi$

Correction en X :

$+2\lambda/\Delta r^2_x$

0    $r_{ox}$    $\sqrt{2}r_{ox}$    X

## FIG_10B

$2\pi$

Correction
en Y

$-2\lambda/\Delta r^2_y$

0    $r_{oy}$    $\sqrt{2}r_{oy}$    Y

FIG_11A

Correction en X

$-2\lambda/\Delta r^2_x$

FIG_11B

Correction
en Y

$+2\lambda/\Delta r^2_y$

FIG_12A

Correction en X :

$+2\lambda/\Delta r^2_x$

FIG_12B

Correction
en Y :

$-2\lambda/\Delta r^2_y$

FIG_13A

Correction en X :
à l'ordre $p = +1$ :
$+ 1/f_x = 2\lambda/\Delta r^2_x$
à l'ordre $p = -1$
$+ 1/f_x = -2\lambda/\Delta r^2_x$

FIG_13B

Correction en Y
à l'ordre $p = +1$
$+1/f_y = +2\lambda/\Delta r^2_y$
à l'ordre $p = -1$
$+1/f_y = -2\lambda/\Delta r^2_y$

FIG_14A

Correction en X
à l'ordre $p = +1$
$+ 1/f_x = + 2\lambda/\Delta r^2_x$
à l'ordre $p = -1$
$+ 1/f_x = -2\lambda/\Delta r^2_x$

FIG_14B

Correction en Y
à l'ordre $p = +1$
$+1/f_y = +2\lambda/\Delta r^2_y$
à l'ordre $p = -1$
$+1/f_y = -2\lambda/\Delta r^2_y$

Correction $+1/f_x = 2\lambda/\Delta r^2_x$

$2\pi$

0

$r_{ox}$   $\sqrt{2}\,r_{ox}$   X

## FIG. 15A

Correction: $+1/f_y = -2\lambda/\Delta r^2 y$

$2\pi$

0

$r_{oy}$   $\sqrt{2}\,r_{oy}$   Y

## FIG. 15B